# EUROPEAN PATENT APPLICATION

(11) **EP 1 878 385 A1**
(43) Date of publication of application: **16.01.2008**
(21) Application number: 06291152.4
(22) Date of filing: 13.07.2006
(51) Int. Cl.: A61B 5/103, A61B 5/0408, A61B 5/091

(54) **Device for collecting physiological information of an animal, and corresponding method**

(71) Applicant: INSTITUT NATIONAL DE LA SANTE ET DE LA RECHERCHE MEDICALE (INSERM), 75013 Paris (FR)
(72) Inventor: Vardon, Guy, Routhiauville, 80120 Quend (FR); Gallego, Jorge, 75020 Paris (FR); Matrot, Boris, 75010 Paris (FR)
(74) Representative: Blot, Philippe Robert Emile

(57) **Abstract**

The device comprises:
- a tight test enclosure (14) for receiving the animal (16),
- a means (20, 21) for thermally regulating the test enclosure (14),
- a means (27) for renewing air in the test enclosure (14) at a controlled flow rate,
- a pressure sensor (41) for measuring pressure difference between the air in the test enclosure and a reference,
- a means (43) for deducing a volume of air inhaled or expired by the animal during one inhalation or expiration from a pressure measurement of the pressure sensor (41),

The test enclosure (14) comprises a floor having at least three dry electrodes, insulated from each other, each electrode being intended for collecting an electrical signal resulting from the cardiac activity of the animal (16) when the animal is in contact with the electrode. The device comprises a means (51) for determining an electrocardiogram of the animal (16) by using three electrical signals collected by the at least three electrodes.

## Description

The invention relates to a device for collecting physiological information of an animal, and a corresponding method.

It is known in the art to collect physiological information about the volume of air inhaled by an animal by using a device of the type that comprises:
- a tight test enclosure for receiving the animal,
- a means for thermally regulating the test enclosure,
- a means for renewing air in the test enclosure at a controlled flow rate,
- a sensor for measuring pressure difference between the air in the test enclosure and a reference,
- a means for deducing a volume of air inhaled or expired by the animal during one inhalation or expiration from a pressure measurement in the test enclosure.

The human genotype being almost entirely known, research is now focused on the determination of the functions of the genes and their implication in human diseases.

To this end, mice constitute a very good modelisation of the human genotype, as they share about 90% of their genotype with the human beings.

Accordingly, genetically modified mice are studied in order to determine a relation between their genetic modification and their phenotype constituted in part by physiological information. The information concerning the volume of air inhaled by a mouse is often determined using the above previous known device.

It is often desirable to measure, at the same time, other physiological information, of course without any disturbance to the air volume measurement. In particular, the electrocardiogram of the animal is a very important physiological parameter. However, no known device is able to achived this.

Furthermore, recent research has focused on new born babies in order to find treatment that is suitable for them. This has lead to a large activity in the study of the phenotype of young mice.

Accordingly, it is an object of the invention to provide a device for simultaneously collecting at least the volume of air inhaled by an animal and its electrocardiogram with a high accuracy, the device further being capable to be used on a young rodent such as a young mouse.

The invention therefore relates to a device of the previous type being characterized in that:
- the test enclosure comprises a floor having at least three dry electrodes, insulated from each other, each electrode being intended for collecting an electrical signal resulting from the cardiac activity of the animal when the animal is in contact with the electrode,
- the device comprises a means for determining an electrocardiogram of the animal by using three electrical signals collected by the at least three electrodes.

Other features of the device are set forth in the dependent claims.

The invention further relates to a method for collecting physiological information of an animal, particularly a small rodent such as a small mouse, the method comprising:
- placing the animal in a closed tight test enclosure for receiving the animal, the enclosure being thermally regulated and the air of the enclosure being renewed at a controlled rate,
- measuring air pressure in the enclosure,
- deducing a volume of air inhaled or expired by the animal during one inhalation or expiration from the pressure measurement,
the method being characterized in that it comprises:
- collecting three electrical signals resulting from cardiac activity of the animal, by using a floor of the enclosure having at least three dry electrodes, insulated from each other, and
- determining an electrocardiogram of the animal by using the three electrical signals collected by the at least three electrodes.

Other features of the method are set forth in the dependent claim.

The invention will be better understood upon reading the following detailed description of a preferred embodiment of the invention, with reference to the accompanying drawings:
- figure 1 is a schematically top view of a system comprising several collecting devices according to the invention;
- figure 2 is a three dimensional view of an enclosure of one of the collecting devices of figure 1;
- figure 3 is a view of the electrical arrangement of a floor on which an animal is intended to be placed;
- figure 4 is a cross sectional view of the floor of figure 3 along line III-III;
- figure 5 is a view similar to figure 3 showing an alternative embodiment of the floor; and
- figure 6 is a three dimensional view of one of the collecting devices of figure 1.

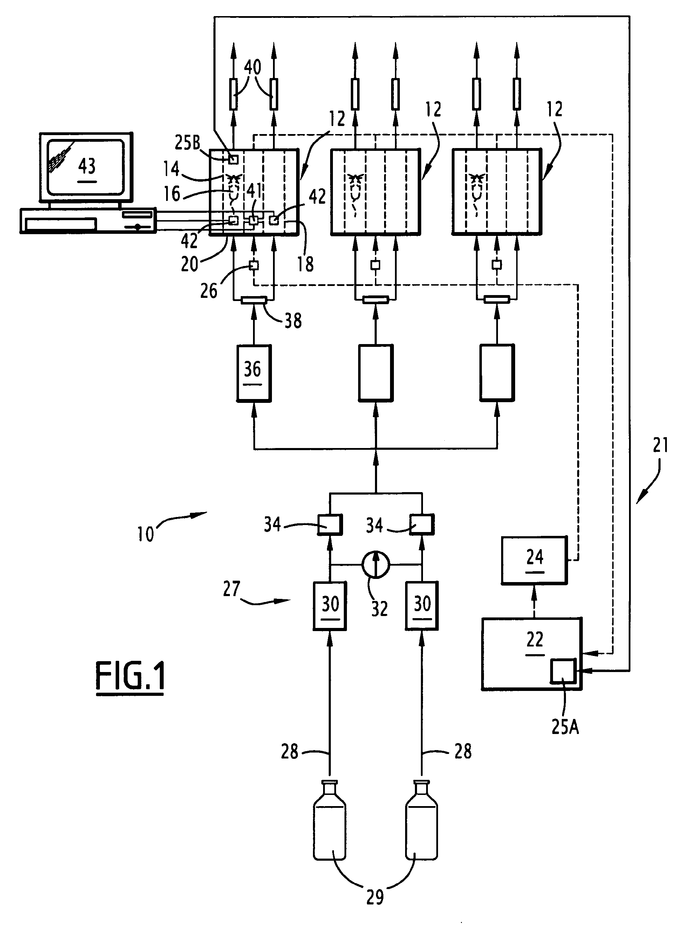

Turning to figure 1, a system 10 for collecting physiological information of several animals comprises a plurality of identical collecting devices 12.

For clarity reason, the references indicated on figure 1 are only indicated on one of the collecting devices 12 and a description will be given for only this collecting device, the others being identical.

The collecting device 12 comprises a tight test enclosure 14 for receiving an animal 16, such as a young mouse, and a reference enclosure 18 intended to remain empty. The test and reference enclosures 14, 18 are of identical dimension.

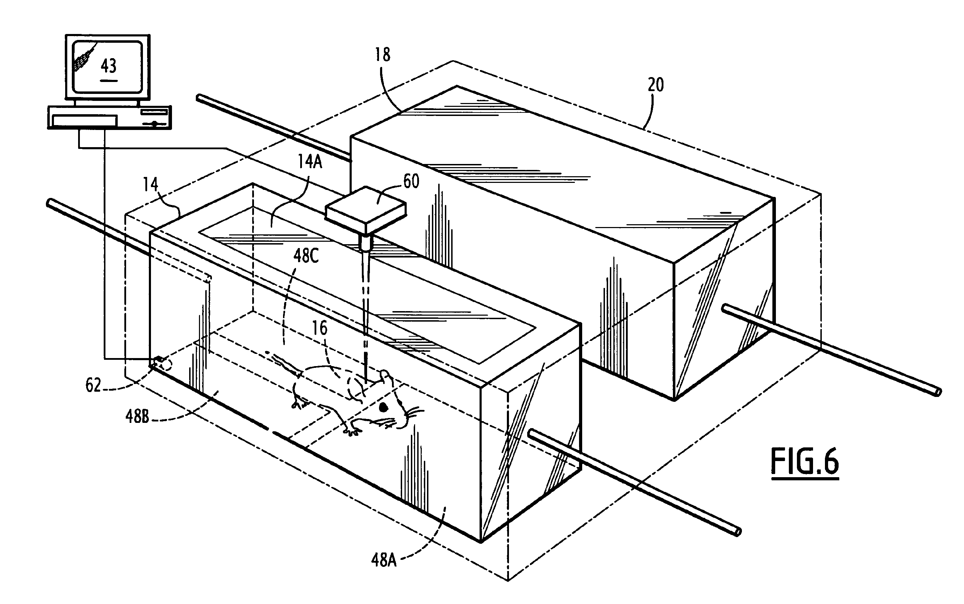

The enclosures 14, 18 are made of transparent Plexiglas, except for at least a window 14A of the test enclosure 14 that will be described later with reference to figure 6. The transparency of the enclosures 14, 18 makes it possible to check the activity of the animal by visual contact or by using a video camera.

The video-camera is for example a webcam (not shown) connected to a computer 43, and placed close to the lateral transparent wall of the test enclosure allowing to observe, classify and quantify the animal movements.

The size of the enclosures 14, 18 is large enough to introduce a young mouse, weighting up to 17 grams. They preferably each delimit a volume of 40 to 90 mliters.

In operation, each enclosure 14, 18 is completely closed except for air renewal pipes as will be explained later.

Both enclosures 14, 18 are placed in a trough 20 intended to be filled up with water in which the test enclosure 14 and the reference enclosure 18 are immerged. In this way, the water creates a sound isolation barrier so that each enclosure 14, 18 forms an anechoical chamber.

The water circulates in a closed loop water circuit 21 between a tank 22 and the trough 20. The water is pumped out from the tank 22 at a rate of 0 to 2 liters per minute by using a pump 24. The water in the tank 22 is heated by appropriate means such as a resistor 25A. A valve 26 regulates the flow of water entering the trough 20 while a sensor 25B controls the temperature of the water in the trough, i.e. by driving the resistor 25A. The water in the trough 20 is maintained in this way at a desired temperature, forming a thermostatic bath.

Air renewal is achieved by using an air circuit 27 comprising entries 28 for at least two gas mixtures, contained in respective bottles 29, each equipped with a pressure reducer 30.

The air circuit further comprises a pressure gauge 32 for regulating the pressure of the gas mixtures to a given pressure.

The air circuit further comprises a valve 34 for each gas mixture, for switching between the different gas mixtures to form air for the enclosures 14, 18. Switching between different gas mixtures allows to assess cardiorespiratory and arousal reflexes to hypoxia, or to hyperoxia, which are crucial markers on neonatal adaptation to extra-uterine life.

The air circuit also comprises a pressure gauge 36 in which the air is introduced, for regulating the air flow rate between 0 to 200 mliters per minute.

The regulated air is equally distributed between the test enclosure 14 and the reference enclosure 18 through an entry resistance 38 formed by capillary tubes. Preferably, the capillary tubes are immerged in the trough 20 so as to bring the air to the temperature of the enclosures 14, 18.

The air is evacuated from the enclosures 14, 18 through respective output resistances 40 each formed by a cone-point set screw.

The air circuit provides an air flow renewal in the enclosures at the rate of 25 to 50 mliters per minute, so as to evacuate the CO₂ and the water vapour exhaled by the animal 16.

The system 10 further comprises a pressure sensor 41 and temperature sensors 42 for each enclosure 14, 18. The pressure sensor 41 is adapted for measuring pressure difference between the air in the test enclosure 14 and a reference.

In the illustrated system, the reference is the air of the reference enclosure 18. The pressure sensor 41 is thus connected to the air of both enclosures 14, 18. Using the air of the reference enclosure 18 as a reference allows the measurement of pressure difference below one 0,1 milli bar.

As an alternative, the pressure sensor 41 could be of a type using an other reference, as the atmospheric pressure or even vacuum (in which case, the pressure sensor is usually call an "absolute" sensor). The precision would though decrease, unless maybe if a sensor of prohibitive cost were used.

The sensors 41, 42 are connected to the computer 43, the computer 43 receiving pressure and temperature measurements.

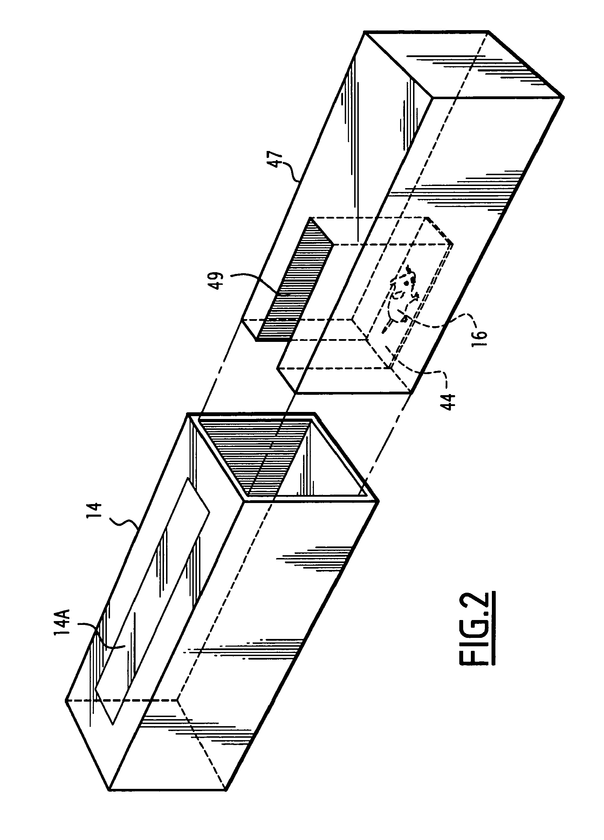

Turning to figure 2, for receiving a smaller mouse, as a newborn mouse weighting about 1 gram, each enclosure is arranged to receive thick and transparent Plexiglas separating walls 47 so as to reduce the volume of the enclosure. The adaptation of the volume of the enclosures 14, 18 as a function of the size of the animal permits to increase the accuracy of the inhaled air measurement. This is illustrated only for the test enclosure 14, the separating walls for the reference enclosure being identical.

The separating walls 47 have a U-shape delimiting a restraint volume 49 between the branches of the U. The separating walls 47 extend until the test enclosure 14 so as to fill the space between the restraint volume 49 and the test enclosure 14.

A floor 44 intended to receive the mouse 16 extend in the restraint volume.

In the absence of separating wall 47, the floor 44 would extend until the test enclosure 14, as illustrated on figure 6.

In order to study very precisely the phenotype of the mouse, the computer is connected to other sensors described in the following, so that the pressure and temperature measurements may be correlated with other physiological measurements.

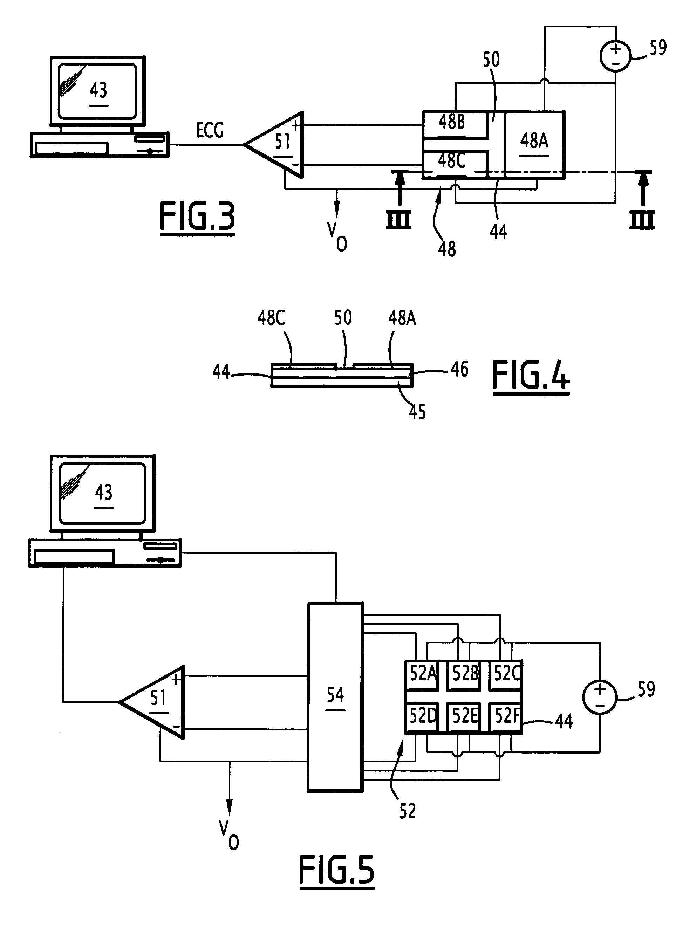

Turning to figures 3 and 4, the floor 44 comprises three layers. A layer of copper 45 is intended to form an electromagnetic shield and a support for an insulating layer 46, on which three dry electrodes 48A, 48B, 48C, made of gold, are disposed. The electrodes are generally referenced with numeral 48. The electrodes 48 form a rectangular surface on which the animal 16 is intended to be in contact with. The electrodes 48 are insulated from each other by an air gap 50.

The first electrode 48A is a reference electrode and extends on approximately a front half of the floor 44, namely from a longitudinal edge of the floor 44 to about the center of the floor 42. The two others electrodes 48B, 48C are measurement electrodes and extend on the remaining surface of the floor 44, i.e. a rear half. More precisely, each measurement electrode 48B, 48C extends from a respective lateral edge of the floor 42 to the half of the floor 44 in the lateral direction, where they meet each other. In this way, the animal 16, will most of the time be in contact with the reference electrode 48A and the two measurement electrodes 48B, 48C.

The electrodes 48 are connected to a differential amplifier 51, which is connected to a reference voltage V0 (ground reference point). The reference electrode 48A is connected to the reference voltage V0, while each measurement electrode 48B, 48C is connected to a respective differential input of the differential amplifier 51, usually called "plus" and "minus". The differential amplifier 51 therefore outputs the voltage difference between the measurement electrodes 48B, 48C with reference to the reference electrode 48A. This output represents a measurement of the electrocardiogram (ECG) of the animal and is sent to the computer 43.

It should be noted that the measurement is realised without any handling of the animal, like the placing of probes. In fact, this kind of handling could not be achieved on a small rodent.

Turning to figure 5, an alternative embodiment of the floor 42 is shown. In this embodiment, the floor 42 comprises six measurement electrodes 52A to 52F disposed in a check-patterned way. The electrodes are generally referenced with numeral 52.

Each electrode 52 is connected to a switching device 54, which is connected to the differential entries of the differential amplifier 51 and to the reference voltage V0. The computer 43 is connected to the switching device 54 so as to detect at least two measurement electrodes contacting the animal 16. The computer 43 is programmed to set the switching device 54 to connect two of these contacting electrodes to the differential entries of the differential amplifier 51, the four remaining electrodes being connected to the reference voltage V0.

The electrodes, either of the embodiment of figure 2, 3 or 4, are also adapted to send electrical stimuli to the animal 16. To this purpose, the system comprises a voltage source 59 connected to the electrodes so as to energise them selectively. The voltage source 59 is controlled by the computer 43.

Turning to figure 6, the window 14A of the test enclosure 14 is placed facing the floor 44, i.e. above the animal 16. The window 14A is made of Zinc Selenide so as to be transparent to infrared radiation emitted by the animal 16. The system 10 comprises a temperature sensor 60 using infrared radiation placed outside the test enclosure 14 The sensor 60 is thus adapted to measure a temperature of the animal 16 by measuring the infrared radiation through the window 14A. The temperature sensor 60 is mobile so as to be orientated toward the animal, through the infrared transparent window 14A of the test enclosure 14. The temperature sensor 60 is connected to the computer 43 for transmitting the temperature measurement.

Furthermore, the system 10 comprises an ultrasonic microphone 62 disposed in the test enclosure 14. Because the test enclosure 14 forms an anechoidal chamber, precise measurement can be achieved. The frequency range of the microphone 62 goes from 30 to 120 kHz so as to be sensitive to ultrasound generated by a small mouse. The microphone 62 does not produce heat or water vapour, or only in unnoticeable quantity, so as it does not disturb the measurement of the volume of air inhaled or expired. The microphone 62 is connected to the computer 43 for transmitting ultrasound measurement.

As described above, the system 10 is configured so that the computer 43 simultaneously receives several measurements, amongst which:
- the pressure and temperature inside the test enclosure,
- the pressure and temperature inside the reference enclosure,
- the electrocardiogram of the animal,
- the ultrasonic sound emitted by the animal, and
- the skin temperature of the animal.

The computer 43 comprises program means (not shown) for processing the received measurements, in order to achieve the operations described below.

Before measurements begin, the air circuit ant water circuit are started off so as to thermally regulate the enclosures 14, 18 and renew the air of the enclosures 14, 18 at a controlled rate and with a controlled composition. The composition of the air is commanded by the computer 43.

An animal such as a young mouse 16 is placed in the test enclosure 14 while the reference enclosure 18 is left empty. The volume for receiving the animal is adapted to the size of the animal, if necessary, by using separating walls 47 in both enclosures 14, 18.

The computer 43 is started off and set to continuously receive the previous measurements.

The computer deduces the volume of air inhaled by the animal from the received pressure measurements. More precisely, the program uses the pressure difference between the air in the test enclosure 14 and the air in the reference enclosure 18, according to the Drorbaugh and Fenn equation.

In a similar way, the computer 43 deduces the volume of air expired by the animal 16.

The computer displays the measurements and the data deduced thereof, so that researchers can process them according to their needs.

An example of operation of the system, using some of the previous measurements, will now be described.

The system of the invention is suitable in particular for studying a new born mouse (until the adolescence of the mouse), which closely compares to a human preterm infant from 19 to 23 weeks of gestational age.

At this age, preterm newborn display respiratory instability characterized by apneas and bradycardias, especially during sleep. This instability, which is often associated with Imparment of the arousal and ventilatory responses to the lack of oxygen, may compromise neurodevelopmental outcome.

In order to determine a genetic predisposition for these developmental disorders, a genetically modified young mouse is studied by using the system of the invention.

The composition of the air is set in the air circuit 27 so that it lacks oxygen.

At the same time, the ECG and volume inhaled are monitored. The EGG gives indication about the sleep stage of the mouse, while the volume inhaled gives indication on respiratory impairments.

In this way, researches can deduce if the genetic abnormalities affect the capacity of the mouse to wake up when it lacks oxygen, according to the sleep stage.

More generally, the measurements received by the computer 43 could be used to improve the phenotype description of the mouse, in the context of any disease of the newborn, including Sudden Infant Death Syndrome, or Apparent Life-Threating event (ALTE), perinatal brain lesions, etc.

## Claims

1. Device for collecting physiological information of an animal (16), in particular a young rodent such as a young mouse, the device comprising:
- a test tight enclosure (14) for receiving the animal (16),
- a means (20, 21) for thermally regulating the test enclosure (14),
- a means (27) for renewing air in the test enclosure (14) at a controlled flow rate,
- a pressure sensor (41) for measuring pressure difference between the air in the test enclosure and a reference ,
- a means (43) for deducing a volume of air inhaled or expired by the animal (16) during one inhalation or expiration from a pressure measurement of the pressure sensor (41),
the device being **characterized in that**:
- the test enclosure (14) comprises a floor (44) having at least three dry electrodes (48; 52), insulated from each other, each electrode being intended for collecting an electrical signal resulting from the cardiac activity of the animal (16) when the animal is in contact with the electrode,
- the device comprises a means (51) for determining an electrocardiogram of the animal (16) by using three electrical signals collected by the at least three electrodes.

2. Device according to claim 1, **characterized in that** the device comprises:
- a reference enclosure (18) of identical dimension to the test enclosure (14), the reference enclosure (18) being intended to remain empty,
- a means (20, 21) for thermally regulating the reference enclosure (18), in the same way as for the test enclosure (14),
- a means (27) for renewing air in the reference enclosure (18) at a controlled flow rate, in the same way as for the test enclosure (14),
wherein the reference of the pressure sensor (41) is the air of the reference enclosure (18).

3. Device according to claim 2, **characterized in that** the means (43) for deducing the volume of air inhaled or exhaled by the animal is configured for using the Drorbaugh and Fenn equation.

4. Device according to any of the preceding claims, **characterized in that** it comprises a means (59) which is connected to at least one electrode (48; 52) so as to send an electrical stimuli to the animal (16) through the electrode (48; 52).

5. Device according to any of the preceding claims, **characterized in that** the means (20, 21) for thermally regulating the test enclosure (14) comprises a thermostatic bath in which the test enclosure is immerged.

6. Device according to any one of the preceding claims, **characterized in that** the test enclosure (14) comprises a window (14A) transparent to infrared radiation.

7. Device according to claim 6, **characterized in that** it comprises a infrared temperature sensor (60) placed outside the test enclosure (14) and adapted to be orientated toward the animal (16) in the test enclosure (14) through the window (14A).

8. Device according to any one of the preceding claims, **characterized** the device comprises an ultrasonic microphone (62) disposed inside the test enclosure (14).

9. Device according to any of the preceding claims, **characterized in that** the test enclosure (14) delimits a volume from 40 to 90 mliters.

10. Method for collecting physiological information of an animal (16), particularly a small rodent such as a small mouse, the method comprising:
- placing the animal (16) in a closed test tight enclosure (14) for receiving the animal, the test enclosure (14) being thermally regulated and the air of the enclosure being renewed at a controlled rate,
- measuring pressure difference between the air in the test enclosure (14) and a reference,
- deducing a volume of air inhaled or expired by the animal (16) during one inhalation or expiration from the pressure measurement,
the method being **characterized in that** it comprises:
- collecting three electrical signals resulting from cardiac activity of the animal (16), by using a floor (44) of the test enclosure (14) having at least three dry electrodes (48; 52), insulated from each other, and
- determining an electrocardiogram of the animal (16) by using the three electrical signals collected by the at least three electrodes (48; 52).

11. Method according to claim 10, **characterized in that** the reference of the pressure measurement is the air of a reference enclosure (18) of identical dimension to the test enclosure (14), the reference enclosure (18) being intended to remain empty and being thermally regulated, in the same way as for the test enclosure (14), the air of the reference enclosure (18) being renewed, in the same way as for the test enclosure (14).

12. Method according to claim 10 or 11, wherein the test enclosure (14) comprises a window (14A) transparent to infrared radiation, **characterized in that** the method comprises measuring a temperature of the animal (16) by measuring infrared radiation emitted by the animal (16) through the window (14A).

13. Method according to any one of claims 10 to 12, **characterized in that** the method comprises measuring ultrasonic sound emitted by the animal (16) placed in the test enclosure (14).

14. Method according to any one of claims 10 to 13, **characterized in that** the method comprises sending an electrical stimuli to the animal (16) placed in the test enclosure (14) through at least one electrode (48; 52).
